# EUROPEAN PATENT APPLICATION

(11) **EP 2 541 450 A1**
(43) Date of publication of application: **02.01.2013**
(21) Application number: 11172416.7
(22) Date of filing: 01.07.2011
(51) Int. Cl.: G06F 19/00, A61B 5/00, G06N 7/00

(54) **Patient mobile computing system and method for exacerbation prediction**

(71) Applicant: Stichting Katholieke Universiteit, 6525 HP Nijmegen (NL)
(72) Inventor: Lucas, Petrus Johannes Franciscus, 3511 MC Utrecht (NL)
(74) Representative: van Westenbrugge, Andries

(57) **Abstract**

Patient mobile computing system (1) and method for predicting exacerbations in a patient illness related status. The method comprises collecting patient sensor data using sensors (8), and executing a local prediction model. The local prediction model comprising a Bayesian network (6) to obtain a probability of an exacerbation of a patient status. The Bayesian network (6) receives patient input data, patient sensor data, and providing probability data, and the local prediction model provides guidance data based on the probability data. The local prediction model (6) is implemented both in a portable device (1), such as a smart phone, and in a centralized patient data processing system (20).

## Description

### Field of the invention

The present invention relates to a patient mobile computing system comprising a sensor interface unit, in communication with at least one sensor unit arranged for measuring patient sensor data, a patient input/output unit, and a processing unit connected to the sensor interface unit and the patient input/output unit.

In a further aspect, the present invention relates to a method for predicting exacerbations in a patient illness related status, comprising collecting patient sensor data.

### Prior art

eHealth and telemedicine are generally defined as the provision of health-care support at a distance from the care centre (hospital and primary-care centre). So far, eHealth and telemedicine solutions are either based on the internet, where a personal computer is used to communicate with the patient or to collect individual measurements, or on the cell (mobile) phone where again individual measurements are transmitted, e.g. as text messages (SMS), to an information system on a central server. Thus, so far, eHealth and telemedicine applications have mainly focused on digital means to measure patient data at home instead of in the clinic, which were then sent to a central server for inspection by the care giver. In this way, the decision-making based on the gathered patient information is still done by healthcare professionals, i.e. clinical specialists and specially trained nurses.

### Summary of the invention

The present invention seeks to provide an improved method and system for allowing patients to be less involved in hospital visits and the like, while still maintaining a sufficient level of care and monitoring.

According to the present invention, a patient mobile computing system according to the preamble defined above is provided, wherein the processing unit is arranged to execute a local prediction model, the local prediction model comprising a Bayesian network to obtain a probability of an exacerbation of a patient illness related status, the Bayesian network receiving patient input data, patient sensor data, and providing probability data, wherein the local prediction model provides guidance data to the patient input/output unit based on the probability data. The present invention is an example of mobile health-care support (mHealth). The patient mobile processing unit using e.g. artificial intelligence techniques, is able to draw conclusions autonomously without a care giver or physician required to be present. When the Bayesian network receives patient input data and patient sensor data as input, posterior probabilities are computed and used for prediction of the current and future health state of the patient. Further questioning of the patient is also based on the probabilistic information represented in the Bayesian network.

In a further aspect, a method as defined above is provided, further comprising executing a local prediction model, the local prediction model comprising a Bayesian network to obtain a probability of an exacerbation of a patient status, the Bayesian network receiving patient input data, patient sensor data, and providing probability data, wherein the local prediction model provides guidance data based on the probability data.

The present invention embodiments allow a patient suffering from various illness (e.g. COPD related illness (Chronic Obstructive Pulmonary Disease), or diabetes) to be monitored remotely (i.e. without presence of a care giver), while still safeguarding timely detection of possible exacerbations in the illness. Furthermore, also illness related issues, syndromes, and the like can be the subject of a specific modeling in the present invention embodiments, such as the (early) detection of pre-eclampsia in pregnant women. Also in this case, a prediction can be made for the development of a disease or syndrome, based on patient input data to questions, and patient sensor input. As a result, timely measures can be taken, such as administering cortisol for patients suffering from COPD, timely hospitalization in case of pre-eclampsia, etc.

### Short description of drawings

The present invention will be discussed in more detail below, using a number of exemplary embodiments, with reference to the attached drawings, in which
Fig. 1 shows a schematic diagram of a system implementing an embodiment of the present invention;
Fig. 2 shows a schematic diagram showing the various users in the context of the present invention embodiments;
Fig. 3 shows a diagram of a Bayesian network according to an embodiment of the present invention relating to prediction of exacerbations in COPD patients;
Fig. 4 shows a diagram of a Bayesian network according to a further embodiment of the present invention relating to pre-eclampsia prediction.

### Detailed description of exemplary embodiments

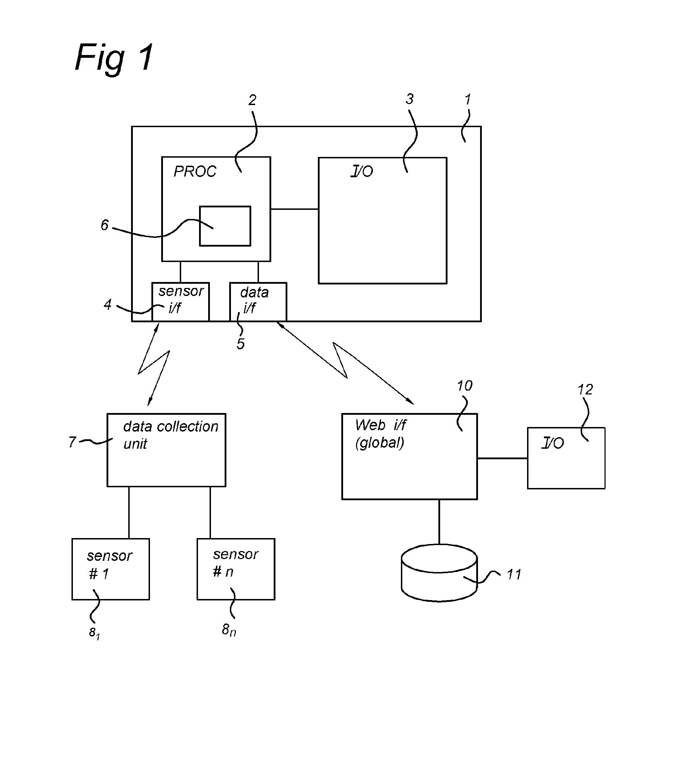

In Fig. 1 a graphical representation of the exacerbation monitoring system-setup is shown, comprising a patient mobile computing system 1, e.g. in the form of a smart phone or other processor based devices which are programmable, such as a tablet PC, laptop computer or PC. The patient mobile computing system 1 comprises a processor 2 able to execute software programs, and to collect and store various patient data in a suitable local database. The software program may be related to a local prediction model 6, e.g. using a Bayesian network as discussed in detail below. Furthermore, the patient mobile computing system 1 comprises an input-output unit 3, e.g. in the form of a touch screen, which is able to present data in a numeric or graphic format to a user, and to receive user input data.

The patient mobile computing system 1 further comprises a sensor interface 4 connected to the processor 2. The sensor interface 4 allows the processor 2 to receive patient sensor data from one or more patient sensors 8₁-8ₙ. Optionally, the interfacing is accomplished using a data collection unit 7. As examples, the sensors 8₁-8ₙ may comprise one or more of the group of a (micro-)spirometer, a pulse-oximeter (SpO₂), thermometer, and a peroxide (H₂O₂) sensor. Furthermore, other devices may be used to obtain actual patient sensor data, e.g. a camera can be used when measuring e.g. creatinine en albumin in urine samples.

Furthermore, in a specific embodiment, the patient mobile computing system 1 comprises a data interface 5. This data interface 5 is e.g. arranged to interface with a web-centre or a (global) web interface (portal) which is located remotely from the patient mobile computing system 1. The web interface unit 10 is e.g. part of a central patient monitoring system in a hospital, and is connected to a memory unit 11 (e.g. a database0 and an input/output unit 12, allowing a physician or assistant (or other health-care workers) to monitor and process patient data for a multiplicity of patients.

Advantageously, the combination of elements described above for the patient mobile computing system 1 is already available in many present day personal computing systems, such as a smart phone or a media player. The sensor interface 4 may e.g. be implemented using Bluetooth technology which is known as such, and the data interface 5 may be implemented using an IP interface for wireless internet (GPRS, UMTS, LTE, etc.).

Data in many forms is collected from the patient through the patient mobile computing system 1, e.g. using wireless communication between the sensor interface 4 and the sensors 8₁-8ₙ (optionally via data collection unit 7). However, also patient input data received from the input/output unit 3 is collected, e.g. in the form of questionnaire replies from the patient.

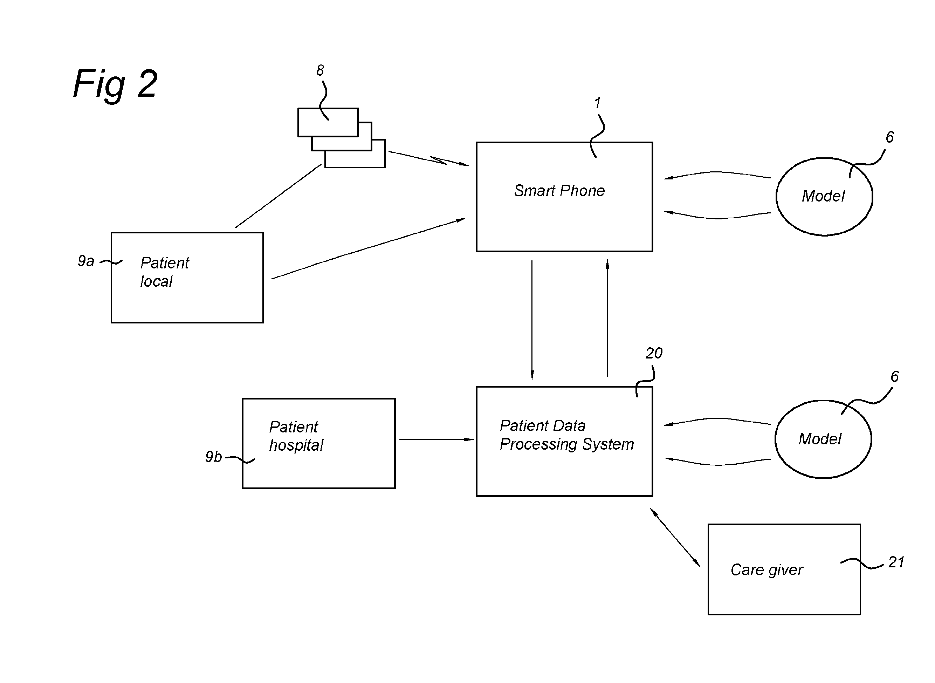

In general wording, the present invention relates to a patient mobile computing system 1 (e.g. smart phone) comprising a sensor interface unit 4 in communication with at least one sensor unit 8₁-8ₙ arranged for measuring patient sensor data, a patient input/output unit 3 (e.g. a touch screen) and a processing unit 2 connected to the sensor interface unit 4 and the patient input/output unit 3. The processing unit 2 is arranged to execute a local prediction model (e.g. under control of a dedicated software program), the local prediction model comprising a Bayesian network to obtain a probability of an exacerbation of a patient illness related status. The Bayesian network, which will be discussed in further detail below with reference to Fig. 2, receives patient input data and patient sensor data, and provides probability data. The local prediction model further provides guidance data (e.g. on sensor use or questionnaire input) to the user input/output unit 3 based on the probability data.

Patient input data can be related to patient characteristics such as age and gender, signs and symptoms, possibly automatically obtained from electronic medical records or from the patient. Patient sensor data corresponds to various data obtained using sensors 8₁-8ₙ, but also to data obtained using tests in a laboratory or hospital environment.

This allows e.g. to implement the present invention embodiment on a smart phone, e.g. using a software program or application in a smart phone using a suitable Operating System (such as Android or iPhone Operating System). In principle any Android phone with Bluetooth capability should suffice, which makes the platform fairly general. The application is custom made to provide the questionnaire functionality, to manage communication with the sensors 8₁-8ₙ and the web-centre 10, and to compute the model predictions using the processor 2.

The Bayesian network 6 used as local prediction model provides an implementation of an algorithm used for learning probabilities from data. For the monitoring application which is executed in the patient mobile computing system 1 a lightweight reasoning engine was used for Bayesian networks. In an exemplary implementation, the Bayesian network 6 is written in Java, and thus the model inference could easily be integrated in the Android application. Due to the relatively small size of the Bayesian network 6 and the processing power of processors 2 of modem smart phones it turns out that the inference does not have to be deferred to a centrally located server but can be performed locally in the smart phone 1. This has the advantage that even when mobile phone network coverage is suboptimal or not present at all, the application can still provide a probability estimate.

In Fig. 2 a typical implementation schematic is shown for using the present invention embodiments. A patient data processing system 20 is shown, which e.g. implements the functionality of the web interface 10 as shown in Fig. 1. In Fig. 2 the main aspect is indicated, i.e. the embedded intelligent Bayesian network 6 in both the patient mobile computing unit 1 and in the patient data processing system 20, which allows for computing the current status of the patient and inform at the same time both the patient and the physician (indicated as 'care giver' 21 able to interface with the patient data processing system 20). Patient input data and patient sensor data can be gathered both locally (indicated as 'Patient local' 9a), i.e. at home or remote form a hospital or laboratory environment, and at a central location (indicated as 'Patient hospital 9b). The Bayesian network 6 is central to the entire application, as it drives the decision support. The system includes a modem smart phone interface (patient input/output unit 3) where the patient can make effective use of a touch screen to enter and obtain information.

In the present invention embodiments, data interpretation for exacerbation detection is performed by a Bayesian network 6.

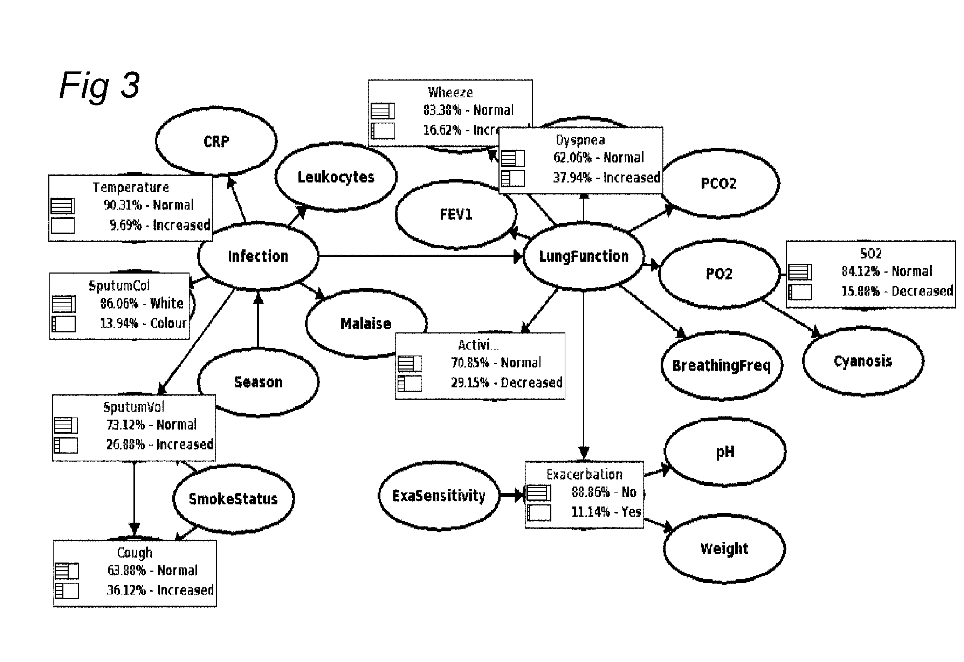

A Bayesian network is a probabilistic graphical model consisting of vertices representing random variables of interest and arcs representing dependencies between variables, as shown in an exemplary embodiment for detecting exacerbations in COPD patients in Fig. 3. Each random variable has a quantitative part, denoting conditional probabilities of the type *P(X* |*pa(X)),* that is the probability that X takes on a specific value given the configuration of its parent variables. From the joint probability over all variables probabilities of interest can be computed, which in this case means the probability of exacerbation given the evidence obtained from monitoring. An important observation is that although the model describes general relations between the variables of interest, all predictions are personalized by entering patient specific data. The model is thus capable of making predictions for individual situations, and can provide 'what-if' -predictions by entering virtual evidence.

In a further embodiment, the Bayesian network 6 is a temporal Bayesian network 6. This embodiment may be applied in diseases or syndromes where the development over time is relevant for making a good prediction relating to exacerbations in a patient's status. For one or more variables in the temporal Bayesian network 6, links between functional status of a variable are established at successive moments in time. This type of Bayesian network 6 is advantageously used in the prediction of pre-eclampsia, which will be discussed in more detail below.

Bayesian networks 6 (and temporal Bayesian networks) are as such known to the person skilled in the art. general information can e.g. be found in the book 'Probabilistic Networks and Expert Systems' by R. Cowelll et al., Springer-verlag, New York, 1999, the contents of which are included herein by reference.

For the embodiment used for prediction of exacerbations in patients suffering from COPD the variables in the Bayesian network 6 are related to patient sensor data and patient input data, the patient sensor data related variables comprising one or more of the group comprising lung function (measured using a spirometer), blood oxygen level (SpO₂ measured using e.g. a pulse-oximeter), and blood gas content (e.g. measured using a peroxide sensor), and the patient input data related variables comprising one or more patient symptom input data from the group of dyspnea, sputum volume, purulence, cough, wheeze, difficulty in performing daily activities.

The main outcome variable of the Bayesian network 6 is 'exacerbation', but the nature of a Bayesian network 6 allows easy inspection of probabilities for any variable. In the implementation shown, the Bayesian network 6 comprises two 'hidden' variables, namely 'infection' and 'lung function' which cannot be observed directly, but whose values can be derived based on indirect measures, such as body temperature for infection and the forced expiratory volume in 1 second (FEV1) for lung function.

Other important variables are the symptoms that one might expect a patient to report, such as dyspnea (breathlessness), sputum volume and purulence, cough, wheeze and whether performing daily activities is difficult due to COPD. These patient input data can be gathered by presenting the patient with suitable questionnaires and receiving the patient input data, e.g. using the patient input/output unit 3.

Additionally some clinical signs have been included such as the aforementioned FEV1, blood oxygen saturation (SpO₂), CRP concentration, leukocytosis and blood gas and pH levels. Except for FEV1 and SpO₂, these variables are mostly included for the sake of completeness, as they will not be observable in a home-care setting.

However, personal patient data may of course be obtained in a central location such as a hospital, and entered into the synchronized patient data database 11. From there, it may be used as patient input data in the patient mobile computing system 1.

The data collection unit 7 which is used in an embodiment of the present invention allows the patient mobile computing system 1 to communicate with the sensors 8, e.g. using Bluetooth capable multichannel sensor interface. In an exemplary embodiment, a pulse-oximeter and a custom micro-spirometer were connected to the data collection unit 7. An important advantage of using an existing sensor interface in the form of data collection unit 7 is the availability of the communication-protocol specification, enabling to integrate the sensor readings seamlessly into the Android application.

The pulse-oximeter used in an embodiment of this invention is a Nonin Medical 8000AA, which is an industry standard pulse-oximeter. SpO₂ accuracy as reported is 70-100% +/- 2 digits. The spirometer used in an embodiment is newly developed to interact with the data collection unit 7 and has the advantage of providing raw data such that analyzing the spirometer readings is possible without requiring external software. This enables tight integration with the application being executed in the patient mobile computing device 1, which is difficult or impossible with most commercial spirometers on the market.

The web interface 10 provides an interface to the gathered data and is used to enroll patients, schedule registrations and similar practical issues. A software program is built using a workflow management system to implement advanced features to generate and coordinate tasks and provides a generic (web)interface. Since the data management involved with patient monitoring is suitable to be represented as a workflow, standard available tools can be used in a simple and effective way to construct the web interface 10.

In general wording, a further embodiment is provided wherein the processing unit 2 is arranged to interface with a remotely located central processing unit 10 via a radio interface unit 5 connected to the processing unit 2, the remotely located central processing unit 10 being arranged to collect data available to the Bayesian network model 6 in the patient mobile computing system 1, and to send instruction data to the processing unit 2 in the patient mobile computing system 1. This can be utilized for synchronization purposes, analysis using further model(s) (or the same Bayesian network 6 as used in the patient mobile computing system 1), analysis of data by a physician or nurse, and for providing guidance to a specific patient (e.g. 'contact physician', 'contact hospital',...).

The monitoring method which can be advantageously implemented using the embodiments of the present invention will now be described in relation to the monitoring process for detecting exacerbations relating to COPD patients. At regular intervals, adjustable in frequency and in time of the day, the patient gets an automatic reminder for data entry from the smart phone 1. The patient is presented with a simple touch-interface to answer a set of questions about COPD symptoms and is subsequently asked to perform a spirometry test, and a pulseoximeter measurement. The results of the measurements are transmitted to the smart phone 1, and entered in a Bayesian network model 6 (described in more detail below) to obtain a probability of exacerbation. In addition, the data is synchronized with the web interface 10, which allows the responsible health-care workers to examine the patient data. Depending on the situation this may be a nurse specialized in lung diseases, a general practitioner or a chest physician. If necessary, the patient can be advised to take action, based on the model's prediction.

In general words, a method for predicting exacerbations in patient illness related status is provided, comprising collecting patient sensor data, executing a local prediction model, the local prediction model comprising a Bayesian network to obtain a probability of an exacerbation of a patient status, the Bayesian network receiving patient input data, patient sensor data, and providing probability data, wherein the local prediction model provides guidance data based on the probability data.

Monitoring patients over longer periods of time requires a careful balance between costs and benefits. Specifically the intrusiveness of monitoring systems and costs both monetary and in terms of patient time investment result in a target Population of patients with moderate to severe COPD and frequent exacerbations. These patients suffer greatly from the consequences of exacerbations, hence providing regular data to detect exacerbations in an early stage will in general be more acceptable. The most appropriate time to start the intervention would be directly after hospitalization due to COPD, because then the goal of preventing hospitalization is clearly relevant for the patient.

Due to the privacy sensitive nature of the data, care has to be taken to properly secure all data transmission. Also access rights to the data in the web interface 10 have to be controlled and patients should give prior consent.

Ease of use is a critical requirement for any system that has to be used on a regular basis for a prolonged period of time. Since the interval between exacerbations is usually in the order of months, one should take care to reduce patient effort to a minimum, lest patients would stop entering data due to it being inconvenient. The patient population is relatively old on average - possibly not very experienced with technology - hence to facilitate understanding the web interface may be arranged to provide the ability to do practice runs. The nurse will have a supportive role in training patients.

Depending on the health status of the individual patient the rate of data acquisition can be varied, this process can be automated based on the acquired data and the Bayesian network model 6. That is, as long as a patient has low risk of an exacerbation, monitoring can e.g. take place on a weekly basis, keeping the time investment at a minimum. If a patient is at risk according to model predictions, the system check-in can be scheduled daily to ensure the possible exacerbation is indeed detected and acted upon appropriately. The system may be arranged to be limited by not implementing specific advise beyond contacting a physician, but self-management supporting advise could also be implemented in further embodiments.

The Bayesian network 6 has been constructed in close cooperation with specialists, and a set of relevant variables that are related to exacerbations has been identified by domain experts - in this case chest physicians - supported with findings from current medical literature. As a second step the dependence relations between the variables have been elicited from the domain experts, resulting in the qualitative part of the model. Subsequently, probabilities have been estimated in two ways. Starting from qualitative constraints we have elicited probabilities from expert opinion and secondly, using a dataset from an earlier project, probabilities were estimated using the expectation-maximization algorithm.

By examining a number of test cases we can show that the model predictions are reasonable. In Table 1 some realistic data entries are shown with the corresponding model prediction of the probability of an exacerbation. Examining these cases will show that the prediction at least follow what one would intuitively expect. For ease of exposition focus was set on a subset of variables that are representative for the behavior of the network model. The first row represents a stable situation with the variables taking on 'normal' values, clearly the probability of exacerbation should be quite close to zero, and indeed it is. The second row shows a situation where dyspnea and sputum volume are observed (say by asking the patient), but oxygen saturation, temperature and FEV1 have not been measured (denoted by a dash in the table). As dyspnea (breathlessness) is increased, the probability of exacerbation also increases, however with only this information the probability of an exacerbation is not very high yet. If a lung function measurement would be performed and found that the FEV1 is decreased (although not dramatically so), it becomes more likely that the patient would have an exacerbation (row 3). In the following situation the patient does not report dyspnea but does report a fever, this increases the probability of exacerbation somewhat, but since fever can be caused by all kinds of non-airway related infections, this finding does not provide a lot of evidence for an exacerbation. Observing that sputum volume is increased makes it more likely that the fever is caused by an airway infection and hence the probability of exacerbation increases. Now consider the situation where a patient reports dyspnea and increased sputum, but does not feel up to testing FEV1 (which is quite strenuous), an oxygen saturation test that turns up a percentage below 92 makes it quite likely that the patient has an exacerbation. The final case shows that if all findings are unfavorable the probability of exacerbation is indeed high, as expected. Note that the outcome is also influenced by the variable 'exacerbation sensitivity', not considered in the table. Taking into account that the model in the form of the Bayesian network 6 will be used for patients that generally have frequent exacerbations, the probabilities would turn out higher. Based on these kind of case studies, the outcomes could be divided in ranges to decide at what probability a certain action (like contacting a chest physician) is warranted. These test cases give some feeling for how the model reacts to certain findings, and the results match intuition quite well.

**Table 1.**

| FEV1 (%decrease) | Dyspnea (Normal/Increased) | SpO₂ (%) | Temperature (°C) | Sputum volume (Normal/Increased) | Exacerbation (%predicted) |
|---|---|---|---|---|---|
| 0 | Normal | >92 | <38.5 | Normal | 2 |
| - | Increased | - | | Normal | 13 |
| -10 | Increased | - | | Normal | 37 |
| - | Normal | - | >38.5 | - | 8 |
| - | Normal | - | >38.5 | Increased | 17 |
| - | Increased | ≤92 | <38.5 | Increased | 65 |
| -10 | Increased | ≤92 | ≥38.5 | Increased | 73 |

In a further aspect, the present invention embodiments are used for prediction of possible exacerbations relating to pre-eclampsia. The design of the Bayesian network 6 has been driven by the consideration that preeclampsia is basically a disorder affecting the normal physiological functioning of the cardiovascular and renal systems; the clinician interprets the condition of the patients in these terms. Preeclampsia is a syndrome rather than a disease.

While a syndrome is described by findings, which by definition are correlated to the syndrome, there need not be a cause-effect relationship between them. The reason is that a syndrome is just a label that summarizes a set of findings, rather than a well-understood disease process that allows explaining the findings in terms of disease mechanisms. Thus, where for a syndrome it is not known (yet) what causes it, the causes of a disease are known. The findings of a syndrome are, however, in the end the result of changes in the physiology of particular organs. Thus, another view on a syndrome is that it summarizes physiological changes of particular organs, which in turn can be determines by means of observation, i.e., by means of a medical interview, physical examination, and laboratory investigations. The physiological changes are partly an effect of risk factors, such as age, gender, genetic constitution; they are also influenced by treatments such as taking drugs, which, for example, may reduce the effect of particular risk factors. The embodiments as described above, may thus also be advantageously used in this case, using patient input data and patient sensor data.

Although a static 'snapshot' of the body's physiology may be sufficient for diagnostic purposes, static information is often insufficient for a reliable prediction of the evolution of a disorder. For the latter, observing the organs' functioning over time is a necessity in order to establish trends and make plausible predictions about the potential development of a syndrome.

The health monitoring of a patient usually constitutes of a number of checkups made at different time points t. As we saw in the syndrome model, the functioning of a particular organ X at a specific moment t, which is influenced by the drugs taken, affects the outcomes of laboratory tests. However, taking into account the functioning of X at the previous checkup t-1, allows the medical doctor to conclude whether or not big changes in the functioning occur. The presence of a particular temporal pattern may then act as the basis for a diagnosis at time t, which in turn may trigger the start of a treatment. Furthermore, given the history and the current status, one can also predict the future development of a disorder or syndrome, i.e., their likely development at time points t' > t.

The design of the Bayesian network 6 for preeclampsia was guided by the structure of the temporal conceptual model described above.

The temporal Bayesian network includes the risk factors and laboratory measurements taken during 10 checkups at 12, 16, 20, 24, 28, 32, 36, 38, 40 and 42 weeks of pregnancy (e.g. stored using the web interface 10). The list of all factors and measurements with their set of values and abbreviated names as used in our model are given in Table 2.

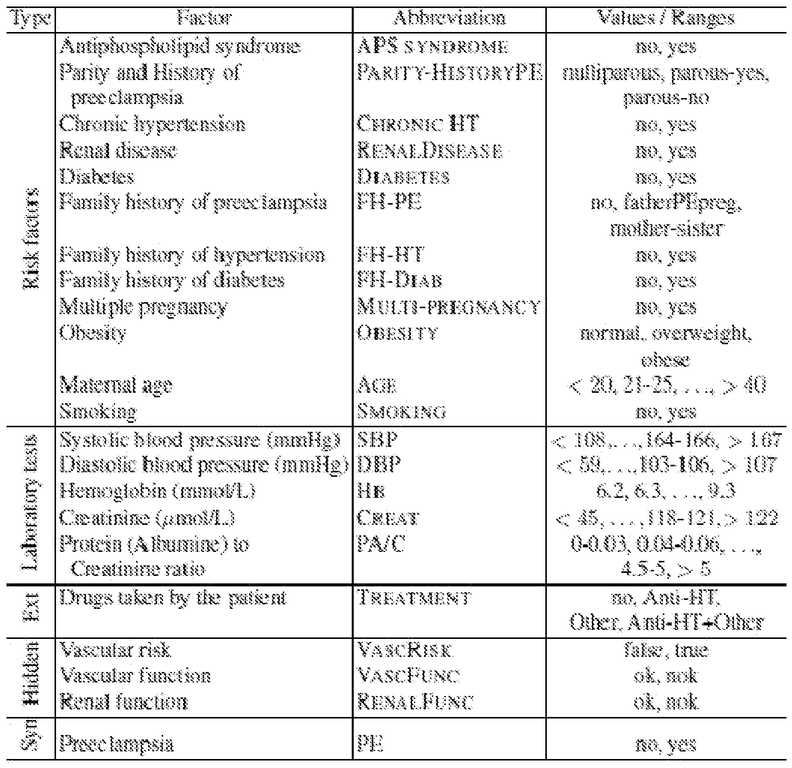

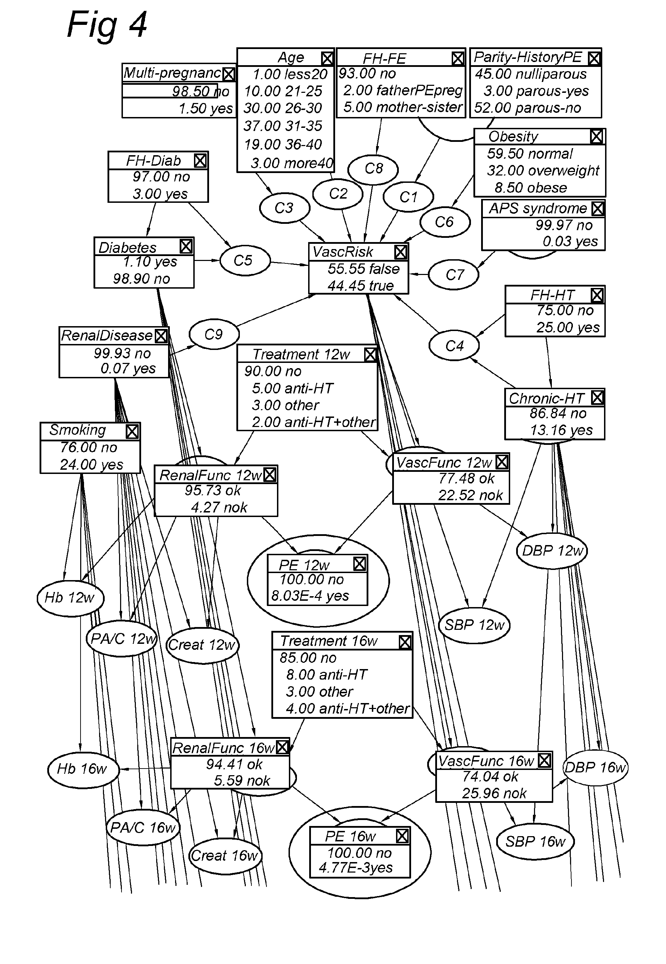

Note that the risk factors 'Parity' and `History of PE' are combined as only parous women can have a history of preeclampsia. The Bayesian network 6 contains 112 variables and part of its structure is depicted in Fig. 4. As discussed earlier the set of risk factors as defined here determines the prior risk for preeclampsia. In particular, these factors have an impact on the functioning of the vascular system and hence, they determine the risk for vascular dysfunction, which for brevity we referred as to vascular risk and in our model it is represented by the hidden variable VASCRISK.

In the current Bayesian network 6, these cause-effect relationships are represented by RISKFACTORᵢ → VASCRISK, i = 1,...,m (with m = 9) structures. Then to define the conditional probability tables (CPT) of VASCRISK given all possible combinations of risk factor values, we need to fill in a table with 20736 entries, which is practically impossible. Therefore to represent in a compact fashion these CPTs, we use causal independence modeling. More precisely, we added the intermediate (hidden, non-observed) variables Cᵢ in order to model the interaction between the risk factors and the vascular risk. Note that the variables FH-DIAB and DIABETES as well as FH-HT and CHRONIC HT influence the vascular risk through a combined effect, represented by one hidden variable. Since, by domain knowledge the risk factors interact by adding up to the vascular risk, we choose the logical OR as a natural function for representing the probabilistic interaction with respect to VASCRISK; this implies that if at least one of the risk factors is present with absolute certainty then the vascular risk is true.

Given the definition of preeclampsia, we consider the renal and vascular functioning as major conditions determining the syndrome. In our modeling scheme these functions are represented as two binary variables with values of *ok* and *nok* (not ok), indicating the respective functional status. Note that these variables are hidden and, thus, cannot be measured. However, as discussed earlier, several laboratory tests are performed at every checkup in order to determine the current health status, including the renal and vascular function, of the patient. The most common and easily made measurements, especially at the beginning of pregnancy, are systolic and diastolic blood pressure reflecting the status of the vascular function and hemoglobin, creatinine and protein (albumin) to creatinine ratio reflecting the status of the renal function. In addition, the values of these measurements are explained by the presence of certain risk factors; for example chronic hypertension affects the blood pressure values, renal disease has an impact on the values of creatinine and protein (albumin) to creatinine ratio, and smoking usually increases the level of hemoglobin. With the exception of the laboratory measurements, however, any treatment taken by the patient at the time of the checkup has an impact on the status of the renal and vascular function, which is captured by the TREATMENT → FUNCTION graph structure.

To capture the temporal development of both renal and vascular functions, we created a temporal model by adding links between the respective functional status at successive week checkups, following the representation in Fig. 4. As a result, the combined status of the renal and vascular function define the development of the syndrome of preeclampsia at each medical checkups. This modeling scheme allows determining the probability for preeclampsia at different stages.

Estimation of the conditional probability distributions for the variables concerned was done as follows. For the risk factors and measurements, we used incidence rates, expert knowledge, and literature studies. The prior probabilities are shown in the Bayesian network 6 as shown in Fig. 4. Time invariance of the conditional probability distributions, i.e., P(Xₜ | Xₜ₋₁) = P(Xᵤ | Xᵤ₋₁), for each time point t, u, was not assumed. The literature clearly indicates that the patient's physiology changes drastically as a function of time, and thus, time invariance is an incorrect assumption in this domain.

Evaluation tests were carried out, and it was noted that already at 12 weeks the present invention embodiments are able to detect that 82% of the preeclamptic patients are suspicious, whereas almost half of the non-preeclamptic patients are judged as being non-suspicious. This is a desired result in patient monitoring where at an early stage a large part of the normal cases would not require intensive checkups thus reducing the workload of the physician and the number of visits to the out-patient clinic by patients.

The present invention embodiments have been described above with reference to a number of exemplary embodiments as discussed and as shown in the drawings. Modifications and alternative implementations of some parts or elements are possible, and are included in the scope of protection as defined in the appended claims.

## Claims

**1.** Patient mobile computing system comprising
a sensor interface unit, in communication with at least one sensor unit arranged for measuring patient sensor data,
a patient input/output unit,
and a processing unit connected to the sensor interface unit and the patient input/output unit,
the processing unit being arranged to execute a local prediction model,
the local prediction model comprising a Bayesian network to obtain a probability of an exacerbation of a patient illness related status,
the Bayesian network receiving patient input data, patient sensor data, and providing probability data,
wherein the local prediction model provides guidance data to the patient input/output unit based on the probability data.

**2.** Patient mobile computing system according to claim 1, wherein the at least one sensor unit comprises one or more from the group of a spirometer, a pulse-oximeter, a thermometer, and a peroxide sensor.

**3.** Patient mobile computing system according to claim 1 or 2, wherein the Bayesian network is arranged to predict exacerbations in a patient suffering from COPD,
the Bayesian network being a probabilistic graphical model comprising vertices representing variables of interest and arcs representing dependencies between the variables of interest, the variables being related to patient sensor data and patient input data,
the patient sensor data related variables comprising one or more of the group comprising lung function, blood oxygen level, blood gas content, breath air,
and the patient input data related variables comprising one or more patient symptom input data from the group of dyspnea, sputum volume, purulence, cough, wheeze, difficulty in performing daily activities.

**4.** Patient mobile computing system according to any one of claims 1-3, wherein patient input data received by the Bayesian network comprises questionnaire input data from the patient via the patient input/output unit.

**5.** Patient mobile computing system according to any one of claims 1-4, wherein the Bayesian network is arranged to receive virtual patient sensor data and virtual patient input data.

**6.** Patient mobile computing system according to any one of claims 1-5, wherein the processing unit is arranged to interface with a remotely located central processing unit via a radio interface unit connected to the processing unit,
the remotely located central processing unit being arranged to collect data available to the Bayesian network model in the patient mobile computing system, and to send instruction data to the processing unit in the patient mobile computing system.

**7.** Patient mobile computing system according to claim 6, wherein the Bayesian network model is also being executed in the remotely located central processing unit.

**9.** Method for predicting exacerbations in a patient illness related status, comprising
collecting patient sensor data,
executing a local prediction model, the local prediction model comprising a Bayesian network to obtain a probability of an exacerbation of a patient status,
the Bayesian network receiving patient input data, patient sensor data, and providing probability data,
wherein the local prediction model provides guidance data based on the probability data.

**10.** Method for predicting exacerbations according to claim 9, wherein the Bayesian network is arranged to predict exacerbations in a patient suffering from COPD,
the Bayesian network being a probabilistic graphical model comprising vertices representing variables of interest and arcs representing dependencies between the variables of interest, the variables being related to patient sensor data and patient input data,
the patient sensor data related variables comprising one or more of the group comprising lung function, blood oxygen level, blood gas content, breath air,
and the patient input data related variables comprising one or more patient symptom input data from the group of dyspnea, sputum volume, purulence, cough, wheeze, difficulty in performing daily activities.

**11.** Method for predicting exacerbations according to claim 9 or 10, wherein the guidance data is related to performing a sensor measurement, the timing interval thereof being determined by the output of the Bayesian network.

**12.** Method for predicting exacerbations according to claim 9, 10 or 11, wherein the guidance data is related to performing a questionnaire to obtain patient input data, wherein the questionnaire is determined by the output of the Bayesian network.

**13.** Method for predicting exacerbations according to any one of claims 9-12, wherein the Bayesian network is arranged to receive virtual patient sensor data and virtual patient input data.

**14.** Method for predicting exacerbations according to any one of claims 9-13, further comprising interfacing with a remotely located central processing unit,
and at the remotely located central processing unit collecting data of a specific patient, and sending guidance data.

**15.** Software program product comprising computer executable instructions, which when loaded on a computing system, provide the computing system with the functionality of one of the claims 9-14.
